# FASCICULE DE BREVET EUROPEEN

(11) **EP 2 713 850 B1**
(45) Date de publication et mention de la délivrance du brevet: **05.07.2017**
(21) Numéro de dépôt: 12724649.4
(22) Date de dépôt: 31.05.2012
(51) Int. Cl.: A61B 3/113, A61F 4/00, G02B 27/00, G06F 3/01

(54) **SYSTÈME COMPORTANT UN OCULOMÈTRE, PROCÉDÉ MIS EN OEUVRE SUR UN TEL SYSTÈME ET PRODUIT PROGRAMME D'ORDINATEUR CORRESPONDANT**
SYSTEM MIT EINEM OKULOMETER, AUF EINEM DERARTIGEN SYSTEM IMPLEMENTIERTES VERFAHREN UND ENTSPRECHENDES COMPUTERPROGRAMMPRODUKT
SYSTEM COMPRISING AN OCULOMETER, METHOD IMPLEMENTED ON SUCH A SYSTEM AND CORRESPONDING COMPUTER PROGRAM PRODUCT

(30) Priorité: 01.06.2011 FR 1101695
(43) Date de publication de la demande: 09.04.2014
(73) Titulaire: Centre National de la Recherche Scientifique (CNRS), 75016 Paris (FR); Université Pierre et Marie Curie (Paris 6), 75005 Paris (FR)
(72) Inventeur: LORENCEAU, Jean, F-75013 Paris (FR)
(74) Mandataire: Regimbeau
(86) Numéro de dépôt international: PCT/EP2012/060309
(87) Numéro de publication internationale: WO 2012/164043

(56) Documents cités:
- EP-A1- 1 219 243
- US-B1- 7 918 558

## Description

### DOMAINE TECHNIQUE GENERAL

L'invention concerne un système comportant un oculomètre pour mesurer des mouvements d'au moins un oeil d'un opérateur.

L'invention concerne également un procédé mis en oeuvre sur un tel système, et un produit programme d'ordinateur correspondant.

### ETAT DE L'ART

On connaît des systèmes comportant un oculomètre pour mesurer des mouvements d'au moins un oeil d'un opérateur, ayant par exemple perdu une motricité de ses membres supérieurs, pour sélectionner des objets proposés sur un afficheur, par exemple pour une communication écrite.

Les objets proposés sur les afficheurs connus sont par exemple les lettres et/ou les chiffres et/ou les commandes d'un clavier, par exemple du type d'un clavier d'ordinateur.

La sélection connue de chaque objet proposé s'effectue par une séquence comportant en général :
- un mouvement de saccade de l'oeil ayant pour cible l'objet à sélectionner, et
- une fixation de l'oeil sur l'objet à sélectionner pendant une durée prédéterminée et/ou un clignement des paupières,
cette séquence étant mesurée par l'oculomètre précité.

Les techniques précédentes présentent cependant des inconvénients.

Elles ne permettent que la sélection d'objets proposés sous une forme prédéfinie, et non pas une génération d'objets sous une forme de tracé personnalisé. En effet, physiologiquement, les mouvements de l'oeil se composent quasi uniquement de mouvements de saccade entre des cibles (de l'ordre de deux à trois mouvements de saccade par seconde), et ne peuvent donc pas être utilisés pour la génération d'objets sous une forme de tracé personnalisé.

En outre, elles exigent une forte concentration et des efforts soutenus de la part de l'opérateur, pour le contrôle des mouvements de saccade.

Par ailleurs, la sélection précitée est lente.

Enfin, les systèmes connus demandent une calibration réitérée et très précise de l'oculomètre, car ils utilisent la direction du regard de l'oeil pour désigner comme cible l'objet à sélectionner. Le document EP1219243A1 divulgue un système pour détecter une démence d'un patient, et ce en vérifiant l'aptitude d'un patient à suivre des yeux des points mobiles. Le document US7918558 divulgue un système destiné à déclencher un nystagmus optocinétique.

### PRESENTATION DE L'INVENTION

L'invention propose de pallier au moins un de ces inconvénients.

A cet effet, on propose selon l'invention un système comportant un oculomètre pour mesurer des mouvements d'au moins un oeil d'un opérateur, caractérisé en ce qu'il comporte en outre un afficheur pour afficher, pour l'oeil, une trame d'au moins un point effectuant un papillotement, de sorte que l'opérateur puisse effectuer au moins un mouvement de poursuite lisse de l'oeil, en regardant l'afficheur en fonctionnement.

L'invention est avantageusement complétée par les caractéristiques suivantes, prises seules ou en une quelconque de leur combinaison techniquement possible :
- l'afficheur comporte au moins un capteur de coordonnées d'une projection d'une direction d'un regard de l'oeil sur l'afficheur, et est adapté pour afficher un repère dans la trame, la position du repère sur l'afficheur étant fonction des coordonnées de la projection de la direction du regard de l'oeil sur l'afficheur ;
- le système comporte une unité de traitement des mouvements de l'oeil de l'opérateur, pour une génération d'au moins un graphique, et/ou d'au moins une lettre, et/ou d'au moins un chiffre, et/ou d'au moins un son, et/ou d'au moins une commande ;
- l'oculomètre est adapté pour mesurer également un diamètre d'une pupille de l'oeil de l'opérateur, l'unité étant adaptée pour moduler le graphique et/ou la lettre et/ou le chiffre et/ou le son et/ou la commande en fonction du diamètre de la pupille de l'oeil de l'opérateur ;
- le système comporte en outre une interface permettant de régler le contraste et/ou la fréquence de papillotement de la trame ;

L'invention concerne également un procédé mis en oeuvre sur un tel système, et un produit programme d'ordinateur correspondant.

L'invention présente de nombreux avantages.

Elle permet à l'opérateur d'effectuer des mouvements de poursuite lisse de l'oeil.

Elle permet une génération d'objets sous une forme de tracé personnalisé, le tracé étant issu d'un mouvement de poursuite lisse de l'oeil de l'opérateur, mesuré par l'oculomètre. Le tracé personnalisé permet la génération
- d'un graphique et/ou
- d'une lettre (ou d'une succession de lettres pour former un mot ou une succession de mots) et/ou
- d'un chiffre (ou d'une succession de lettres pour former un nombre) et/ou
- d'un son et/ou
- d'une commande.

L'opérateur n'est donc plus limité à des objets ayant une forme prédéfinie, mais peut laisser libre cours à sa créativité, et générer des objets empreints de sa personnalité (comme par exemple une écriture cursive personnelle et/ou une signature), notamment dans un souci d'authentification de l'opérateur.

En outre, elle exige une concentration et un temps d'apprentissage relativement faibles, et s'appuie sur les acquis de génération d'objets par formation manuelle d'une écriture cursive par exemple.

Elle exige des efforts relativement faibles de la part de l'opérateur pour les mouvements de l'oeil, et un repère affiché sur l'afficheur peut permettre d'augmenter encore le contrôle des mouvements de poursuite lisse de l'oeil, pour un confort maximal pour l'opérateur.

En outre, la génération des objets est rapide, pour un débit de communication rapide également.

Enfin, l'invention demande une calibration peu précise, car elle utilise les mouvements relatifs de l'oeil pour former l'objet à générer, et non pas la direction du regard de l'oeil pour désigner comme cible l'objet à sélectionner comme dans l'art antérieur.

L'invention a de nombreux champs d'application. On peut ainsi citer, à titre d'exemples non limitatifs :
- une communication, éventuellement artistique, par écriture cursive et/ou sonore par un opérateur, notamment par un opérateur ayant perdu la motricité de ses membres supérieurs (mais pas la motricité oculaire bien entendu) ;
- une étude et une correction éventuelle des mouvements oculaires, par exemple pour un opérateur présentant des troubles visuels (dégénérescence maculaire liée à l'âge (ou DMLA) par exemple), cognitifs (dyslexie, dyspraxie) ou neurologiques ; ou
- une communication dans des situations où seul au moins un oeil est disponible (opération chirurgicale ou situation de combat pour un pilote d'avion ou un fantassin par exemple).

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 représente schématiquement un mode de réalisation possible d'un système selon l'invention,
- la figure 2 représente schématiquement une mise en oeuvre possible d'un procédé selon l'invention,
- la figure 3 représente schématiquement l'affichage d'une trame sur un afficheur,
- les figures 4A et 4B représentent schématiquement un papillotement d'une trame sur un afficheur, et
- les figures 5A, 5B, 5C et 5D représentent schématiquement des exemples d'objets générés par un système selon l'invention.
Sur l'ensemble des figures, les éléments similaires portent des références numériques identiques.

### DESCRIPTION DETAILLEE

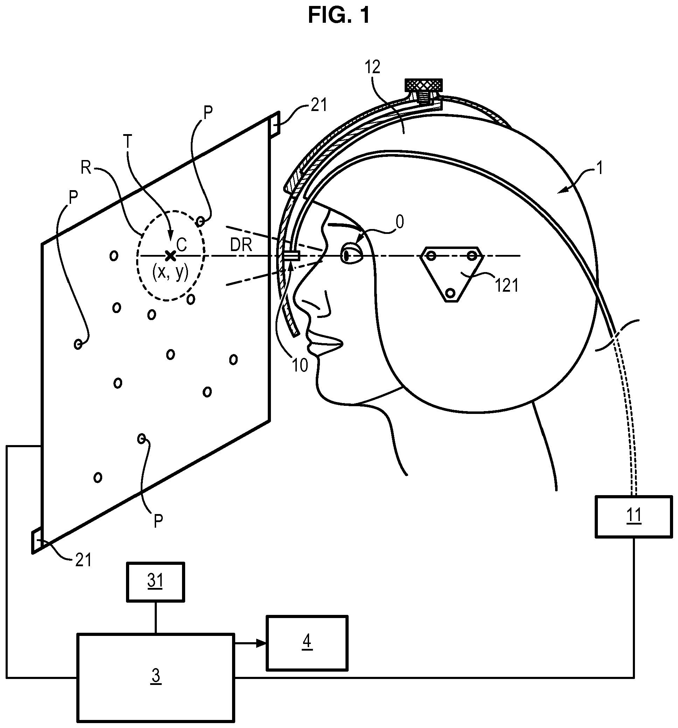

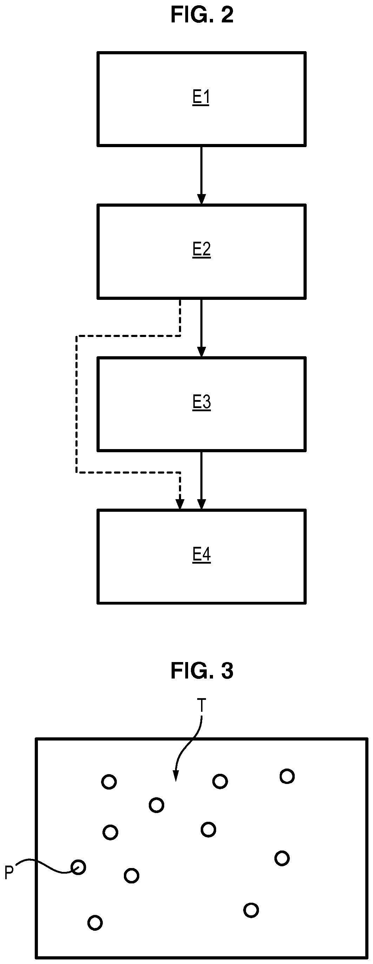

La figure 1 représente schématiquement un mode de réalisation possible d'un système selon l'invention, mettant en oeuvre un procédé possible représenté schématiquement sur la figure 2.

Le système comporte principalement un oculomètre 1, pour mesurer, lors d'une étape E1, des mouvements d'au moins un oeil O d'un opérateur.

L'oculomètre 1 est un appareil de mesure des mouvements de l'oeil, connu de l'homme du métier, et disponible dans le commerce.

Il comporte généralement au moins une caméra 10 orientée vers l'oeil O de l'opérateur, pour une observation directe de l'oeil O et une mesure des mouvements de l'oeil O dans une orbite de l'opérateur.

La caméra 10 est reliée à un processeur 11 de l'oculomètre 1, pour l'enregistrement et le traitement des mouvements de l'oeil O de l'opérateur. Elle est portée par un casque 12 sur l'opérateur.

Un exemple non limitatif d'oculomètre 1 pouvant être utilisé est l'oculomètre de la marque Eye-Link II, de l'entreprise dénommée SR-Research.

Comme le montrent les figures 1, 3, 4A et 4B, le système comporte en outre un afficheur 2 pour afficher, pour l'oeil O et lors d'une étape E2, une trame T constituée d'au moins un point P effectuant un papillotement.

La trame T est un motif quelconque constitué d'au moins le point P, visible par l'opérateur. Préférentiellement, la trame T comporte une pluralité de points P.

Les points P peuvent par exemple être distribués aléatoirement sur l'afficheur 2. Les points P peuvent être de tailles inégales entre elles, et la trame T peut avoir plusieurs densités de points P sur l'afficheur 2.

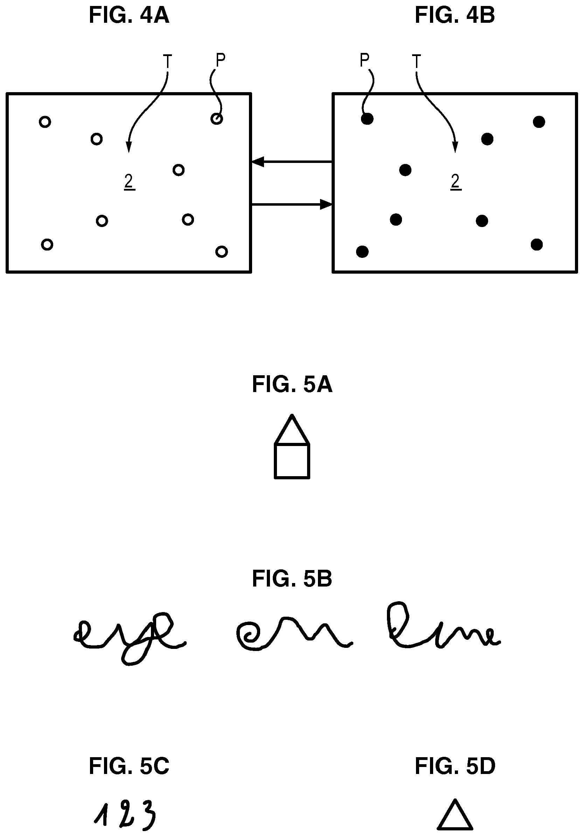

Les figures 4A et 4B montrent plus précisément que la trame T effectue un papillotement sur l'afficheur 2, visible par l'opérateur.

On appelle papillotement un scintillement de la trame T, perceptible par l'oeil O, sur l'afficheur 2.

La trame T est faiblement contrastée par rapport à un fond d'écran de l'afficheur 2, pour éviter une fatigue excessive de l'oeil O de l'opérateur.

Le fond d'écran peut par exemple être de couleur gris moyen, mais toute autre couleur peut bien entendu être envisagée.

Le papillotement est obtenu par inversion cadencée d'une nuance des points de la trame T. Ainsi,
- la trame T est affichée avec une nuance claire (par exemple en blanc ou en une nuance plus claire que la couleur du fond d'écran de l'afficheur 2) pendant une période de temps prédéterminée, par l'afficheur 2, comme le montre la figure 4A ; puis
- la trame T est affichée avec une nuance sombre (par exemple en noire ou en une nuance plus sombre que la couleur du fond d'écran de l'afficheur 2) pendant la même période de temps prédéterminée, par l'afficheur 2, comme le montre la figure 4B.

Et ainsi de suite.

La fréquence de papillotement est ainsi par exemple comprise entre 10Hz et 40Hz, de préférence comprise entre 20Hz et 30Hz, avec une valeur préférentielle à 25Hz par exemple.

L'afficheur 2 peut être un moniteur d'ordinateur ou de téléviseur, ou un écran passif sur lequel la trame T est projetée par un projecteur par exemple.

Le système comporte une unité 3 de traitement (préférentiellement mais non limitativement sous la forme d'un processeur d'un ordinateur), reliée au moniteur ou reliée au projecteur précités, et qui permet de contrôler l'affichage de la trame T sur l'afficheur 2. L'unité 3 de traitement contrôle en outre une interface 31 (par exemple sous la forme d'un écran graphique de contrôle) du système, l'interface 31 permettant à l'opérateur de régler le contraste et/ou la fréquence de papillotement de la trame T.

L'opérateur peut effectuer au moins un mouvement de poursuite lisse de l'oeil O, tout en regardant l'afficheur 2 en fonctionnement, c'est-à-dire affichant le papillotement de la trame T. Un mouvement de poursuite lisse est un mouvement continu de l'oeil dans l'orbite, et non pas un mouvement par saccade.

En effet, le papillotement de la trame forme un stimulus visuel perceptible par l'oeil O et permettant un tel mouvement de poursuite lisse, ce dernier étant impossible en l'absence de papillotement (seuls les mouvements de saccade sont en effet effectués en l'absence de papillotement). Ce phénomène a été constaté expérimentalement, et l'on peut par exemple se référer à Sato, T. (1989), Reversed apparent motion with random dot patterns, Vision Research, 29, 1749-1758, ou à Spillmann L. et al. (1997), Reversed visual motion and self-sustaining eye oscillations, Perception, 26(7) :823-30.

Les mouvements de poursuite lisse de l'oeil O sont mesurés en permanence et en temps réel par l'oculomètre 1 : le processeur 11 enregistre et traite les mouvements de l'oeil O mesurés par la caméra 10.

Comme le montre la figure 1, le processeur 11 est relié à l'unité 3 de traitement, par exemple sous la forme d'un port Ethernet lorsque l'unité 3 de traitement est sous la forme d'un processeur d'un ordinateur.

Les mouvements de l'oeil sont interprétés comme une trajectoire par l'unité 3, et enregistrés sur une mémoire de l'unité 3 de traitement. La trajectoire est restituée sous la forme d'une trace graphique qui peut être traitée par l'unité 3.

Le traitement des mouvements de l'oeil O comporte une prise en compte d'une séparation de la trace en différents segments successifs. La séparation peut être commandée par l'opérateur par exemple soit par un arrêt des mouvements de l'oeil pendant une durée prédéterminée soit par un clignement des paupières de l'oeil.

Comme le montre l'ensemble des figures 5A à 5D, l'unité 3 permet ainsi, lors d'une étape E4, une génération, à partir des segments de la trace graphique :
- d'au moins un graphique (figure 5A), et/ou
- d'au moins une lettre (figure 5B), et/ou
- d'au moins un chiffre (figure 5C), et/ou
- d'au moins un son (non représenté sur les figures), et/ou
- d'au moins une commande pour un dispositif (figure 5D).

L'unité 3 peut être reliée à un dispositif 4, prenant en compte la génération issue de l'unité 3.

Le dispositif 4 peut être de tout type, comme par exemple un ordinateur comportant
- un logiciel de traitement de texte et/ou de dessin et
un écran et/ou une imprimante et/ou une table traçante et/ou une mémoire ; et/ou
- un logiciel de génération de son et un haut-parleur ; et/ou
- un logiciel de domotique relié à des équipements domestiques, comme par exemple des stores, des lampes, etc.

Le graphique de la figure 5A peut être tout graphique, figuratif (ici symbolisant une maison) ou abstrait, ce qui permet notamment une communication et une expression artistique de l'opérateur, par affichage sur l'écran et/ou impression sur l'imprimante et/ou tracé sur la table traçante du dispositif 4.

Comme expliqué ci-dessus, les mots de l'expression « eye on line » de la figure 5B sont séparés les uns des autres par exemple par un clignement des paupières, et chaque mot correspond à un segment de la trace graphique. La figure 5C montre qu'il est également possible de générer le nombre 123 par des chiffres 1, 2 et 3 séparés les uns des autres par exemple par un clignement des paupières.

L'expression de la figure 5B ou le nombre de la figure 5A peuvent affichés sur l'écran et/ou imprimés sur l'imprimante et/ou tracés sur la table traçante et/ou enregistrés sur la mémoire du dispositif 4.

Dans le cas où le dispositif 4 est un ordinateur comportant un logiciel de traitement de texte et/ou de dessin, le traitement par l'unité 3 comporte également une réduction et/ou une mise en forme et/ou une mise en page des segments de la trace graphique. Les mouvements de poursuite lisse de l'oeil O en regardant l'afficheur 2 sont en effet d'une amplitude importante, pour un plus grand confort de mouvement de l'oeil O, et ne correspondent donc en général pas à un format classique, comme un format A4 en mode portrait.

Le traitement précité peut également être effectué en tout ou en partie par le dispositif 4.

Dans le cas où le dispositif comporte également un logiciel de génération de son et un haut-parleur, le dispositif 4 peut également émettre un son modulé, par exemple dans l'intensité et/ou la hauteur et/ou le timbre, en fonction de l'objet généré par les mouvements de l'oeil O.

Dans le cas où le dispositif comporte en combinaison le logiciel de traitement de texte et/ou de dessin et le logiciel de génération de son et un haut-parleur, le dispositif 4 peut également émettre un son correspondant à une lettre générée par les mouvements de l'oeil O.

La figure 5D montre enfin qu'il est également possible de générer une commande « vers le haut », par exemple pour la levée d'un store d'une fenêtre relié au dispositif 4.

La figure 1 montre que l'afficheur 2 comporte au moins un capteur 21 de coordonnées (x,y) d'une projection C d'une direction DR d'un regard de l'oeil O sur l'afficheur 2, cette mesure du capteur 21 étant effectuée pendant une étape E3. A cet effet, le capteur 21 peut ainsi mesurer la position notamment de marques 121 du casque 12, et utiliser les mesures de l'oculomètre 1, car le capteur 121 est relié à l'unité 3.

Le capteur 21 permet ainsi une calibration de l'oculomètre 1 par rapport à l'afficheur 2. Cependant la calibration peut être relativement peu précise, et donc être effectuée rapidement lors d'une initialisation du système, la calibration n'étant pas utilisée pour la génération des objets, cette dernière utilisant principalement les mouvements de poursuite lisse de l'oeil O.

L'afficheur 2 est en outre avantageusement adapté pour afficher, lors de l'étape E3, un repère R dans la trame T, la position du repère R sur l'afficheur 2 étant fonction des coordonnées (x,y) de la projection C de la direction DR du regard de l'oeil O sur l'afficheur 2. Le repère R forme ainsi un indicateur en temps réel, perceptible par l'opérateur, permettant à ce dernier un contrôle du déplacement et de la vitesse de son oeil O.

Le repère R peut être de toute forme, mais est très préférentiellement périphérique par rapport à la projection C de la direction DR du regard de l'oeil O sur l'afficheur 2.

Il est par exemple constitué de disques (ayant par exemple un diamètre comparable à celui des points P) périphériques et formant une couronne circulaire centrée autour de la projection C. Le diamètre de la couronne peut être quelconque, mais représente avantageusement environ entre 1/16^{e} et 1/8^{e} de la surface de l'afficheur 2 par exemple.

Le repère R peut être affiché en permanence lors du fonctionnement de l'afficheur 2, mais peut également être clignotant.

Le repère doit être perceptible à l'oeil, et est par conséquent plus contrasté que la trame T par rapport au fond d'écran de l'afficheur 2.

La forme, la taille et le contraste du repère peuvent être réglés par l'opérateur via l'interface 31 du système.

Selon une variante avantageuse, l'oculomètre 1 est adapté pour mesurer également un diamètre d'une pupille de l'oeil O de l'opérateur, l'unité 3 est adaptée pour moduler le graphique et/ou la lettre et/ou le chiffre et/ou le son et/ou la commande en fonction du diamètre de la pupille de l'oeil O de l'opérateur.

L'épaisseur du trait du tracé peut ainsi augmenter lorsque le diamètre de la pupille augmente, pour augmenter l'expressivité de l'écriture, car le diamètre de la pupille de l'oeil traduit les émotions de l'opérateur.

On comprend que
- tout ou partie du processeur 11 peut être compris dans l'unité 3, et inversement, et/ou
- tout ou partie du dispositif 4 peut être compris dans l'unité 3, et inversement, et/ou
- tout ou partie de l'interface 31 peut être compris dans l'afficheur 2, et inversement.

L'invention concerne également un produit programme d'ordinateur comportant des instructions qui, une fois chargées sur une mémoire d'un ordinateur, permettent la mise en oeuvre d'un procédé selon l'invention. Le produit peut être sur tout support informatique, comme par exemple mémoire ou un CD-Rom.

## Revendications

1. Système comportant un oculomètre (1) pour mesurer des mouvements d'au moins un oeil (O) d'un opérateur, un afficheur (2) pour afficher, pour l'oeil (O), une trame (T) d'au moins un point (P) effectuant un papillotement, de sorte que l'opérateur puisse effectuer au moins un mouvement de poursuite lisse de l'oeil (O), en regardant l'afficheur (2) en fonctionnement.

2. Système selon la revendication 1, dans lequel l'afficheur (2)
- comporte au moins un capteur (21) de coordonnées (x,y) d'une projection (C) d'une direction (DR) d'un regard de l'oeil (O) sur l'afficheur (2), et
- est adapté pour afficher un repère (R) dans la trame (T), la position du repère (R) sur l'afficheur (2) étant fonction des coordonnées (x,y) de la projection (C) de la direction (DR) du regard de l'oeil (O) sur l'afficheur (2).

3. Système selon l'une des revendications 1 ou 2, comportant une unité (3) de traitement des mouvements de l'oeil (O) de l'opérateur, pour une génération :
- d'au moins un graphique, et/ou
- d'au moins une lettre, et/ou
- d'au moins un chiffre, et/ou
- d'au moins un son, et/ou
- d'au moins une commande.

4. Système selon la revendication 3, dans lequel l'oculomètre (1) est adapté pour mesurer également un diamètre d'une pupille de l'oeil (O) de l'opérateur, l'unité (3) étant adaptée pour moduler le graphique et/ou la lettre et/ou le chiffre et/ou le son et/ou la commande en fonction du diamètre de la pupille de l'oeil (O) de l'opérateur.

5. Système selon l'une des revendications 1 à 4, comportant en outre une interface (31) permettant de régler le contraste et/ou la fréquence de papillotement de la trame (T).

6. Procédé comportant une étape selon laquelle un oculomètre (1) mesure (E1) des mouvements d'au moins un oeil (O)d'un opérateur,
une étape (E2) selon laquelle un afficheur (2) affiche, pour l'oeil (O), une trame (T) d'au moins un point (P) effectuant un papillotement, de sorte que l'opérateur puisse effectuer au moins un mouvement de poursuite lisse de l'oeil (O), en regardant l'afficheur (2) en fonctionnement.

7. Procédé selon la revendication 6, comportant une étape selon laquelle au moins un capteur (21) de l'afficheur (2) mesure (E3) des coordonnées (x,y) d'une projection (C) d'une direction (DR) d'un regard de l'oeil (O) sur l'afficheur (2),
et selon laquelle l'afficheur (2) affiche (E3) un repère (R) dans la trame (T), la position du repère (R) sur l'afficheur (2) étant fonction des coordonnées (x,y) de la projection (C) de la direction (DR) du regard de l'oeil (O) sur l'afficheur (2).

8. Procédé selon l'une des revendications 6 ou 7, comportant une étape selon laquelle une unité (3) pour le traitement des mouvements de l'oeil (O) de l'opérateur génère (E4) :
- au moins un graphique, et/ou
- au moins une lettre, et/ou
- au moins un son, et/ou
- au moins une commande.

9. Procédé selon la revendication 8, comportant une étape selon laquelle l'oculomètre (1) mesure également un diamètre d'une pupille de l'oeil (O) de l'opérateur, et selon laquelle l'unité (3) module le graphique et/ou la lettre et/ou le son et/ou la commande en fonction du diamètre de la pupille de l'oeil (O) de l'opérateur.

10. Programme d'ordinateur comportant des instructions qui, une fois chargées sur une mémoire d'un ordinateur d'un oculomètre selon l'une des revendications 1 à 5, permettent la mise en oeuvre d'un procédé selon l'une des revendications 6 à 9.

## Patentansprüche

1. System, umfassend einen Eyetracker (1), um die Bewegungen von mindestens einem Auge (O) eines Bedieners zu messen, eine Anzeige (2), um für das Auge (O) ein Raster (T) von mindestens einem Punkt (P) anzuzeigen, das ein Flimmern durchführt, so dass der Bediener mindestens eine gleichmäßige Folgebewegung des Auges (O) durchführen kann, indem er die Anzeige (2) in Betrieb betrachtet.

2. System nach Anspruch 1, wobei die Anzeige (2)
- mindestens einen Sensor (21) von Koordinaten (x, y) einer Projektion (C) einer Richtung (DR) eines Blicks des Auges (O) auf die Anzeige (2) umfasst, und
- ausgelegt ist, um eine Markierung (R) im Raster (T) anzuzeigen, wobei die Position der Markierung (R) auf der Anzeige (2) eine Funktion der Koordinaten (x, y) der Projektion(C) der Richtung (DR) des Blicks des Auges (O) auf die Anzeige (2) ist.

3. System nach einem der Ansprüche 1 oder 2, umfassend eine Einheit (3) zur Behandlung der Bewegungen des Auges (O) des Bedieners für eine Erzeugung von:
- mindestens eines Diagramms und/oder
- mindestens eines Buchstabens, und/oder
- mindestens einer Zahl, und/oder
- mindestens eines Tons, und/oder
- mindestens eines Befehls.

4. System nach Anspruch 3, wobei der Eyetracker (1) ausgelegt ist, um auch einen Durchmesser einer Pupille des Auges (O) des Bedieners zu messen, wobei die Einheit (3) ausgelegt ist, um das Diagramm und/oder den Buchstaben und/oder die Zahl und/oder den Ton und/oder den Befehl je nach dem Durchmesser der Pupille des Auges (O) des Bedieners zu modulieren.

5. System nach einem der Ansprüche 1 bis 4, umfassend außerdem eine Schnittstelle (31), die ermöglicht, den Kontrast und/oder die Frequenz des Flimmerns des Rasters (T) einzustellen.

6. Verfahren, umfassend einen Schritt, nach dem ein Eyetracker (1) Bewegungen von mindestens einem Auge (O) eines Bedieners misst (E1), einen Schritt (E2), nach dem eine Anzeige (2) für das Auge (O) ein Raster (T) mit mindestens einem Punkt (P) anzeigt, das ein Flimmern durchführt, so dass der Bediener mindestens eine gleichmäßige Folgebewegung des Auges (O) durchführen kann, indem er die Anzeige (2) in Betrieb betrachtet.

7. Verfahren nach Anspruch 6, umfassend einen Schritt, nach dem mindestens ein Sensor (21) der Anzeige (2) Koordinaten (x, y) einer Projektion (C) einer Richtung (DR) eines Blicks des Auges (O) auf die Anzeige (2) misst (E3),
und nach dem die Anzeige (2) eine Markierung (R) im Raster (T) anzeigt (E3), wobei die Position der Markierung (R) auf der Anzeige (2) eine Funktion der Koordinaten (x, y) der Projektion(C) der Richtung (DR) des Blicks des Auges (O) auf die Anzeige (2) ist.

8. Verfahren nach einem der Ansprüche 6 oder 7, umfassend einen Schritt, nach dem eine Einheit (3) zur Behandlung der Bewegungen des Auges (O) des Bedieners Folgendes erzeugt (E4):
- mindestens ein Diagramm und/oder
- mindestens einen Buchstaben, und/oder
- mindestens einen Ton, und/oder
- mindestens einen Befehl.

9. Verfahren nach Anspruch 8, umfassend einen Schritt, nach dem der Eyetracker (1) auch einen Durchmesser einer Pupille des Auges (O) des Bedieners misst, und nach dem die Einheit (3) das Diagramm und/oder den Buchstaben und/oder den Ton und/oder den Befehl je nach dem Durchmesser der Pupille des Auges (O) des Bedieners moduliert.

10. Computerprogramm eines Computers, umfassend Anweisungen, die, nachdem sie in einem Speicher eines Computers eines Eyetrackers nach einem der Ansprüche 1 bis 5 abgelegt sind, die Ausführung eines Verfahrens nach einem der Ansprüche 6 bis 9 ermöglichen.

## Claims

1. System comprising an oculometer (1) for measuring movements of at least one eye (O) of the operator, a display (2) for displaying, for the eye (O), a frame (T) of at least one isolated point (P) performing a blinking, so that the operator can carry out at least one smooth tracking motion of the eye (O) while looking at the display (2) in operation.

2. System according to claim 1, wherein the display (2)
- comprises at least one sensor (21) of coordinates (x,y) of a projection (C) of a direction (DR) of a gaze of the eye (O) onto the display (2), and
- is designed to display a reference point (R) in the frame (T), the position of the reference point (R) on the display (2) depending on the coordinates (x,y) of the projection (C) of the direction (DR) of the gaze of the eye (O) into the display (2).

3. System according to one of claims 1 or 2, comprising a unit (3) for processing the movements of the eye (O) of the operator, for generating:
- at least one graphic, and/or
- at least one letter, and/or
- at least one numeral, and/or
- at least one sound, and/or
- at least one command.

4. System according to claim 3, wherein the oculometer (1) is designed to also measure a diameter of the pupil of the eye (O) of the operator, the unit (3) being designed to modulate the graphic and/or the letter and/or the numeral and/or the sound and/or the command depending on the diameter of the pupil of the eye (O) of the operator.

5. System according to one of claims 1 to 4, also comprising an interface (31) allowing adjustment of the contrast and/or the blinking frequency of the frame (T).

6. Method comprising a step wherein an oculometer (1) measures (E1) movements of at least one eye (O) of the operator, a step (E2) wherein a display (2) displays, for the eye (O), a frame (T) of at least one isolated point (P) performing blinking, such that the operator can carry out at least one smooth tracking motion of the eye (O) while looking at the display (2) in operation.

7. Method according to claim 6, comprising a step wherein at least one sensor (21) of the display (2) measures (E3) coordinates (x,y) of a projection (C) of a direction (DR) of a gaze of the eye (O) onto the display (2),
and wherein the display (2) displays (E3) a reference point (R) in the frame (T), the position of the reference point (R) on the display (2) depending on the coordinates (x,y) of the projection (C) of the direction (DR) of the gaze of the eye (O) onto the display (2).

8. Method according to one of claims 6 or 7, comprising a step wherein a unit (3) for processing movements of the eye (O) of the operator generates (E4) :
- at least one graphic, and/or
- at least one letter, and/or
- at least one sound, and/or
- at least one command.

9. Method according to claim 8, comprising a step wherein the oculometer (1) also measures a diameter of a pupil of the eye (O) of the operator, and wherein the unit (3) modulates the graphic and/or the letter, and/or the sound and/or the command depending on the diameter of the pupil of the eye (O) of the operator.

10. Computer program comprising instructions which, once loaded into a memory of a computer of an oculometer according to one of claims 1 to 5, allow the implementation of a method according to one of claims 6 to 9.
